# EUROPÄISCHE PATENTSCHRIFT

(11) **EP 2 329 860 B1**
(45) Veröffentlichungstag und Bekanntmachung des Hinweises auf die Patenterteilung: **02.09.2015**
(21) Anmeldenummer: 10190653.5
(22) Anmeldetag: 10.11.2010
(51) Int. Cl.: A61N 1/375

(54) **Anschlussgehäuse und dessen Herstellung**
Connection housing and manufacture of same
Boîtier de raccordement et sa fabrication

(30) Priorität: 03.12.2009 US 266172 P
(43) Veröffentlichungstag der Anmeldung: 08.06.2011
(73) Patentinhaber: Biotronik CRM Patent AG, 6341 Baar (CH)
(72) Erfinder: Litzke, Jan, 13507, Berlin (DE); John von Freyend, Jörg, 13125, Berlin (DE)
(74) Vertreter: Galander, Marcus

(56) Entgegenhaltungen:
- EP-A1- 1 795 226
- US-A- 5 620 476
- US-A1- 2001 034 543
- US-A1- 2007 111 587
- US-A1- 2007 255 332
- US-B1- 6 192 277

## Beschreibung

Die Anmeldung betrifft ein Anschlussgehäuse für ein medizinisches Implantat (auch Header genannt), dessen Herstellverfahren und ein medizinisches Implantat mit einem solchen Anschlussgehäuse.

Ein Anschlussgehäuse (auch Header genannt) wird benötigt, um Elektrodenleitungen mit elektrischen Komponenten eines medizinischen Implantats zu verbinden. In der Regel besteht ein solches Anschlussgehäuse aus einem Anschlusskörper aus isolierendem, vorzugsweise transparenten, Material, welches ein Kunststoffmaterial sein kann, vorzugsweise umfasst das Material Polyurethan (PU), Polycarbonat (PC) und/oder Epoxidharz. In diesem Anschlusskörper ist wenigstens eine von außen zugängliche Kavität vorgesehen, in der jeweils Kontaktmittel vorgesehen sind, um die genormten Steckern (wie IS-1, DF-1 oder IS-4) der Elektrodenleitungen aufzunehmen. Bei diesen Elektrodenleitungen kann es sich um Herzelektrodenleitungen, welche intrakardial oder als CS-Elektrode implantiert werden können, um Nerven- oder Epikardelektrodenleitungen handeln. Weiterhin können solche Elektrodenleitungen jede andere Form und Funktion haben.
Die Kontaktmittel in den Kavitäten sind als Federhülsen und/oder als Steckeraufnahmen ausgeführt, in die der Stecker der Elektrodenleitung eingeführt werden kann und welche in Form und Lage den genormten Steckern (wie IS-1, DF-1 oder IS-4) entsprechen. Aufgrund der möglichen Transparenz des Anschlussgehäuses ist von außen sichtbar, ob ein Elektrodenleitungsstecker weit genug in die Federhülse oder die Steckeraufnahme eines jeweiligen elektrischen Anschlusses eingeführt ist.
Die elektrischen Kontaktmittel sind jeweils mit einem oder mehreren elektrischen Leitern verbunden, welche dazu vorgesehen sind, elektrische Signale auf diese elektrischen Kontaktmittel zu übertragen, damit diese über den Elektrodenleitungsstecker zur korrespondierenden Elektrode am distalen Ende der Elektrodenleitung und damit zur Behandlungsstelle im menschlichen oder tierischen Körper geleitet werden können. Genauso können auch im Körper gemessene elektrischen Signale in entgegen gesetzter Richtung geleitet werden. Aus dem Stand der Technik ist bekannt, dass diese elektrischen Leiter aus elektrisch leitendem Metall bestehen, vorzugsweise aus Metalldrähten oder Metallbändern. Diese werden entweder bei der Montage geformt oder als vorgeformtes Band aus einem Blechband (in Nutzen) gestanzt und mittels Fügeverfahren wie Schweißen, Löten oder Crimpen elektrisch mit den elektrischen Kontaktmitteln verbunden. Eine solche bekannte Lösung zeigt die WO 01/99239 A2, deren Offenbarung in dieser Patentanmeldung vollständig einbezogen ist.
Das Anschlussgehäuse kann einen Basiskörper und/oder einen Anschlusskörper umfassen, die in geeigneter Weise miteinander verbunden sind. Es ist auch jeglicher anderer aus dem Stand der Technik bekannter Aufbau möglich, insbesondere ein mehrteiliger Aufbau, bei dem die beinhalteten Komponenten einzeln zusammengefügt sind. Im Folgenden wird daher nur noch von Anschlussgehäuse gesprochen.

Die Erfindung betrifft weiterhin ein medizinisches Implantat wie beispielsweise einen implantierbaren Herzschrittmacher, Defibrillator, Kardioverter, Nervenstimulator oder dergleichen. Das medizinische Implantat umfasst einen eingangs beschriebenen Anschlusskörper oder Header, der an einem Hohlgehäuse befestigt ist. Das Hohlgehäuse ist hermetisch gegen die Umgebung abgedichtet und in der Regel aus biokompatiblem Metall gefertigt. Es dient der Aufnahme einer elektrischen Schaltung oder Steuerelektronik, welche unter anderem aus Kondensatoren, Batterien und anderen elektrischen Schaltungsmitteln besteht. Die Schaltung ist dazu vorgesehen, Signale des menschlichen Körpers zu messen und/oder Signale an den menschlichen Körper abzugeben und sie ist weiterhin geeignet, Signale an einen außerhalb des Körpers liegenden Empfänger zu senden.
Die elektrische Verbindung der elektrischen Schaltung in dem Hohlgehäuse zu den im Header befindlichen elektrischen Leitern, welche mit den elektrischen Kontaktmitteln elektrisch verbunden sind, erfolgt in der Regel über gefilterte oder ungefilterte hermetische Durchführungen, auch bekannt als Glas- / Keramik-Gehäusedurchführungen. Solche Durchführungen sind im Stand der Technik beispielsweise aus der DE 103 29 261 A1, der EP 1 148 910 A1 oder der EP 1 897 589 A2 bekannt. Die Durchführungen bestehen aus mindestens einem Durchführungspin und sind so in eine Wand des Hohlgehäuses eingelassen, dass sie das Hohlgehäuse hermetisch dicht abschließen. Sie können andererseits auch als Filterdurchführungen ausgebildet sein, wobei das Filter eine elektrische Tiefpasswirkung hat. Eine weitere Ausfuehrung einer elektrischen Durchführung ohne Durchführungspin ist aus der US 5,620,476 bekannt.
An die Qualität eines Gehäuses für ein medizinisches Implantat werden hohe Anforderungen gestellt. Insbesondere müssen Hohlgehäuse und Anschlussgehäuse über Jahre hinweg zuverlässig und dicht zusammenwirken. Das Anschlussgehäuse selbst muss über lange Zeit stabil und passgenau sein.
Um die vorgenannten Anforderungen zu erfüllen, sind aus dem Stand der Technik verschiedene Ansätze bekannt. Zum einen ist es bekannt, das Anschlussgehäuse an das Hohlgehäuse direkt anzugießen. Dazu wird das Hohlgehäuse mit an den Durchführungspins durch ein Fügeverfahren befestigte Leiter für die elektrischen Anschlüsse und die elektrischen Kontaktmittel in eine Gussform eingesetzt und durch Füllen der geschlossenen Gussform mit flüssigen Kunststoff umgossen. Der flüssige Kunststoff wird in der Gussform aushärten gelassen und ergibt im Ergebnis - nach Entfernen der Gussform - ein in einem Arbeitsschritt hergestelltes, unmittelbar fest mit dem Hohlgehäuse verbundenes Anschlussgehäuse. Ein anderer Ansatz, der aus den Patentanmeldungen EP 1 795 225 A1 oder EP 1 795 226 A1 bekannt ist, sieht vor, das Anschlussgehäuse separat zu fertigen, indem es zuerst wie soeben genannt in einer Gussform mit den elektrischen Leitern und den elektrischen Kontaktmitteln umgossen und anschließend die Zuleitungen mittels eines Fügeverfahren elektrisch mit den Durchführungspins der Durchführungen im Hohlgehäuse verbunden wird. Zusätzlich wird das Anschlussgehäuse an das Hohlgehäuse mit einem geeigneten flüssigen oder pastenförmigen Klebstoff angeklebt.

Sowohl die Durchführungspins als auch die elektrischen Kontaktmittel sind vor dem Gussvorgang mittels eines Fügeverfahrens elektrisch leitend mit dem elektrischen Leiter verbunden. Ein solches Fügeverfahren umfasst nicht abschließend eine gelaserte Verbindung, Verschweißung, Verlötung oder Verklebung mit einem elektrischen leitfähigen Klebstoff.

Die Herstellung eines Anschlussgehäuses der vorgenannten Art ist nicht risikolos. Beispielsweise können Fehler durch die weitgehende manuelle Fertigung der Anschlussgehäuse entstehen. Zwar können die elektrischen Leiter (Bänder oder Biegestanzteile) in drei Dimensionen vorgebogen gefertigt werden, die Weiterverarbeitung ist jedoch nicht automatisiert möglich. So müssen die elektrischen Leiter und die Kontaktmittel von Hand verschweißt oder verlötet werden und anschließend umgossen werden. Dadurch entstehen ungewollte Fehler, beispielsweise bei der elektrischen Kontaktierung der Kontaktmittel mit dem Leiter.
Weiterhin ist eine Qualitäts- und Funktionskontrolle des Anschlussgehäuses erst dann möglich, wenn das Anschlussgehäuse komplett hergestellt ist. Bei Auftreten von geringen Fehlern ist somit das komplette Anschlussgehäuse nicht mehr brauchbar.

Somit besteht der Bedarf nach einem Anschlussgehäuse oder einem medizinischen Implantat, welches automatisiert hergestellt werden kann und welches vor der endgültigen Herstellung einfach auf Qualität und Funktion getestet werden kann, ohne dass eine komplette Montage erfolgen muss.

Eine Ausführungsform der Erfindung umfasst ein verbessertes Anschlussgehäuse für ein medizinisches Implantat, welches einen Anschlusskörper aus elektrisch isolierendem Material, der wenigstens eine von außen zugängliche Kavität zum Anschließen einer implantierbaren Elektrodenleitung besitzt, Kontaktmittel, die sich in der von außen zugänglichen Kavität befinden und ein aus isolierendem Material bestehendes plattenförmiges Substrat mit mindestens einer elektrisch leitenden Leiterbahnstruktur umfasst, wobei ein oder mehrere Kontaktmittel mit einer oder mehreren der mindestens einen in oder auf dem Substratmaterial des Substrats befindlichen Leiterbahnstruktur fest elektrisch leitend verbunden sind. Der Gegenstand der Patentanmeldung zeichnet sich dadurch aus, dass die Kontaktmittel mit dem Substratmaterial und / oder mit der Leiterbahnstruktur mechanisch fest verbunden sind und das Substrat so in den Anschlusskörper eingebettet ist, dass sich die Kontaktmittel in der von außen zugänglichen Kavität befinden.

Ein Substrat ist im Sinne der Anmeldung als (Schaltkreis-)Platine (auch als Leiterplatte, Printed Circuit Board (PCB), gedruckte Schaltung oder Leiterkarte bekannt) ausgelegt. Eine (Schaltkreis-)Platine ist im Sinne dieser Anmeldung ein Träger für elektronische Bauteile wie Widerstände, Spulen, Kondensatoren und dergleichen. Sie dient der mechanischen Befestigung und elektrischen Verbindung. Das Substrat besteht dabei im Normalfall aus einem isolierenden, nicht transparenten Material, welches üblicherweise faserverstärkte Kunststoffe, Keramik, flammhemmende Substrate aus Papier wie FR1-/FR2-Substrate (Phenolharz und Papier) oder FR3-Substrate (Epoxidharz und Papier), flammhemmende Substrate aus Glasfasergewebe wie, FR4-/FR5-Substrate (Epoxidharz und Glasfasergewebe, BT-Substrat, CE- Substrat, Epoxidharz-Laminate wie CEM1- oder CEM3-Substrate PD-Substrate, PTFE-Substrat (Polytetrafluorethylen), CHn- Substrat, Polyimidfolien (Kaptonfolien), Polyester, oder HDI-Leiterplatte (High-Density-Interconnect-Leiterplatte) ist. Weiter umfasst das Substrat am oder im Substratmaterial befindliche, leitende Verbindungen (Leiterbahnstrukturen), welche zumeist aus leitendem Material wie Kupfer, Aluminium, Gold, Silber, Edelstahl, Titan, Niob, Tantal oder leitfähigen Klebstoffen bzw. Kunststoffen bestehen. Die Leiterbahnstrukturen selbst werden dabei aus einer dünnen Schicht der soeben genannten Materialien geätzt, oder auf das Substrat werden ausgestanzte, geätzte Leiterbahnstrukturen geklebt. Die Bauteile werden auf Lötflächen (Pads) oder in Lötaugen gelötet. So werden sie gleichzeitig mechanisch gehalten und elektrisch verbunden. Im Übrigen sei erwähnt, dass die Leiterbahnstrukturen draht- oder bandförmig sind.

Mit dieser Anordnung ist die komplette Innenverdrahtung des Anschlussgehäuses vormontierbar. Das heißt, das Substrat kann mittels eines geeigneten Designs vor dem Zusammenbau des Anschlussgehäuses gefertigt und ebenfalls in diesem Zustand vor Montage oder Guss des kompletten Anschlussgehäuses getestet werden. Dies verringert den Materialaufwand und erhöht die Zuverlässigkeit der Anschlussgehäuse.

Gemäß einer weiteren Ausführungsform der Erfindung ist ein Verfahren zur Herstellung eines Anschlussgehäuses für ein medizinisches Implantat, welches einen Anschlusskörper aus elektrisch isolierendem Material, der wenigstens eine von außen zugängliche Kavität zum Anschließen einer implantierbaren Elektrodenleitung besitzt, Kontaktmittel, die sich in der von außen zugänglichen Kavität befinden und ein aus isolierendem Material bestehendes plattenförmiges Substrat mit mindestens einer elektrisch leitenden Leiterbahnstruktur umfasst. Ein oder mehrere Kontaktmittel sind mit einer oder mehreren der mindestens einen in oder auf dem Substratmaterial des Substrats befindlichen Leiterbahnstruktur fest elektrisch leitend verbunden und mit dem Substratmaterial und / oder mit der Leiterbahnstruktur mechanisch fest verbunden, wobeidas Substrat so in den Anschlusskörper eingebettet ist, dass sich die Kontaktmittel in der von außen zugänglichen Kavität befinden. Das Verfahren umfasst die folgenden Schritte:
- Fixieren eines elektrischen Substrats mit darauf elektrisch fest verbundenen Kontaktmitteln in einer Spritzgussform,
- Spritzvorgang/Umspritzvorgang des Anschlusskörpers so, dass das Substrat so in den Anschlusskörper durch Umspritzen oder Umgießen so eingebettet ist, dass sich die Kontaktmittel in der von außen zugänglichen Kavität befinden.

Zusätzlich kann als weiterer Verfahrensschritt zwischen Fixierschritt und Spritz-/Umspritzvorgang unmittelbar auf das Substrat eine weitere vom Anschlusskörpermaterial unterschiedliche Passivierungs- bzw. Isolationsschicht aufgebracht werden. Dies kann beispielsweise eine hochspannungsisolierende Beschichtung zwischen Anschlussgehäuse und Substrat sein. Diese Passivierungs- bzw. Isolationsschicht kann aus organischen bzw. anorganischen Werkstoffen und deren Gemischen (beispielsweise Keramiken oder Polymere) bestehen.

So kann der gesamte Fertigungsprozess optimiert und beschleunigt werden, da die Montage des Anschlussgehäuses und des medizinischen Implantats insgesamt mit weniger Fertigungsschritten erfolgt. Die Beschleunigung erfolgt in hohem Maße dadurch, dass dem Montageprozess des Anschlussgehäuses und des medizinischen Implantates das fertig vormontierte und getestete Substrat zugeführt wird. Umfangreiche Verdrahtung und Testung während der Endmontage kann also vermieden werden.

Gemäß einer weiteren Ausführungsform der Erfindung ist ein medizinisches Implantat umfasst, welches umfasst:
- ein Hohlgehäuse, welches hermetisch abgedichtet ist und mindestens eine hermetische Durchführung aufweist, welche geeignet ist, elektrische Signale durch das Hohlgehäuse hindurch zu leiten, ohne dessen Hermetizität zu beeinträchtigen, wobei die hermetische Durchführung mindestens einen Durchführungspin aufweist,
- eine im Hohlgehäuse befindliche Schaltung, wobei die Schaltung geeignet ist, Signale des menschlichen Körpers zu messen und Signale an den menschlichen Körper abzugeben und die geeignet ist, Signale an einen außerhalb des Körpers liegenden Empfänger zu senden, und
- ein Anschlussgehäuse, welches fest mit dem Hohlgehäuse verbunden ist und welches einen Anschlusskörper aus elektrisch isolierendem Material, der wenigstens eine von außen zugängliche Kavität zum Anschließen einer implantierbaren Elektrodenleitung besitzt, Kontaktmittel, die sich in der von außen zugänglichen Kavität befinden und ein aus isolierendem Material bestehendes plattenförmiges Substrat mit mindestens einer elektrisch leitenden Leiterbahnstruktur umfasst, und ein oder mehrere Kontaktmittel, welche mit einer oder mehreren der mindestens einen in oder auf dem Substratmaterial des Substrats befindlichen Leiterbahnstruktur fest elektrisch leitend verbunden und welche mit dem Substratmaterial und / oder mit der Leiterbahnstruktur mechanisch fest verbunden sind, wobei das Substrat ist so in den Anschlusskörper eingebettet ist, dass sich die Kontaktmittel in der von außen zugänglichen Kavität befinden.

Dabei ist jeder Durchführungspin der Durchführung im Hohlgehäuse ist mit einer der mindestens einen elektrisch leitenden Leiterbahnstrukturen des Anschlussgehäuses elektrisch verbunden ist.

Weitere Vorteile oder Aspekte des erfindungsgemäßen Anschlussgehäuses, des Herstellungsverfahrens oder des medizinischen Implantates werden aus den folgenden Zeichnungen und der Beschreibung der Zeichnungen offensichtlich.

Es zeigen:
- Fig. 1A: ein medizinisches Implantat gemäß einer ersten Ausführung
- Fig. 1B: ein Substrat für ein medizinisches Implantat gemäß der ersten Ausführung
- Fig. 2A: ein medizinisches Implantat gemäß einer anderen Ausführung, bei der das Substrat in einer Ebene mit dem Hohlkörper liegt
- Fig. 2B: eine Detailansicht des Substrates gemäß der anderen Ausführung mit Verbindung zum Hohlkörper
- Fig. 3A und Fig. 3B: Schnittansichten eines Substrates für das Anschlussgehäuse eines medizinischen Implantates, die die verschiedenen Leiterbahnstrukturen zeigen
- Fig. 4: eine Detailansicht eines mehrlagigen Substrates für das Anschlussgehäuse eines medizinischen Implantates
- Fig. 5A und Fig. 5B: eine Detailansicht eines medizinischen Implantates gemäß einer weiteren Ausführung
- Fig. 6A und Fig. 6B: eine Detailansicht einer weiteren Verankerungsmöglichkeit vom Substrat an den Hohlkörper

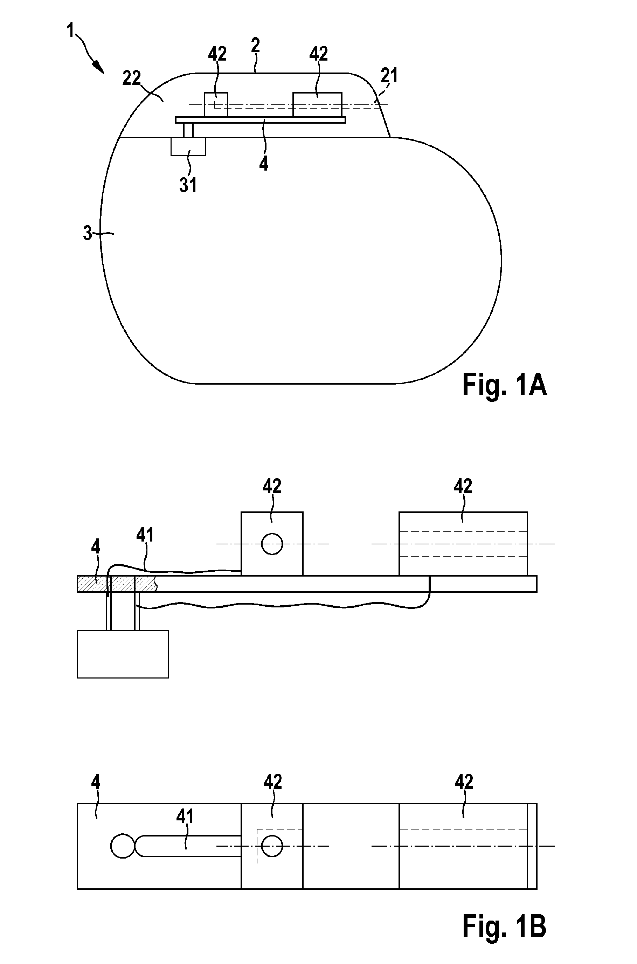

Fig. 1A zeigt ein medizinisches Implantat 1 gemäß einer ersten Ausführung mit einem verbesserten Anschlussgehäuse 2 und einem Hohlgehäuse 3. Das Hohlgehäuse 3 ist hermetisch abgedichtet und in der Regel aus biokompatiblem Metall wie Titan gefertigt. Weiterhin weist es mindestens eine hermetische Durchführung 31 - die auf dem Gehäuse oder im Gehäuse eingebettet ist - auf, welche geeignet ist, elektrische Signale durch das Hohlgehäuse hindurch zu leiten, ohne dessen Hermetizität zu beeinträchtigen. Die hermetische Durchführung weist mindestens einen Durchführungspin auf.
Das Hohlgehäuse 3 dient der Aufnahme einer Schaltung oder Steuerelektronik, welche unter anderem aus Kondensatoren, Batterien und anderen elektrischen Schaltungsmitteln besteht. Die Schaltung ist dafür vorgesehen und geeignet, Signale des menschlichen Körpers zu messen und Signale an den menschlichen Körper abzugeben und sie ist geeignet, Signale an einen außerhalb des Körpers liegenden Empfänger zu senden.
Das Anschlussgehäuse 2 umfasst einen Anschlusskörper 22 aus elektrisch isolierendem Material, vorzugsweise transparenten Material, welches ein Kunststoffmaterial sein kann, vorzugsweise umfasst das Material Polyurethan (PU), Polycarbonat (PC) und/oder Epoxidharz. Weiterhin umfasst das Anschlussgehäuse 2 wenigstens eine im Anschlusskörper 22 von außen zugängliche Kavität 21 zum Anschließen einer nicht dargestellten implantierbaren Elektrodenleitung und ein ebenfalls in den Anschlusskörper 22 eingebettetes, aus isolierendem Material bestehendes, Substrat 4 mit mindestens einer elektrisch leitenden Leiterbahnstruktur 41. Ein oder mehrere Kontaktmittel 42, sind mit einer oder mehreren der mindestens einen in oder auf dem Substratmaterial des Substrats 4 befindlichen Leiterbahnstruktur 41 fest elektrisch leitend verbunden und das Substrat ist so in den Anschlusskörper 22 eingebettet, dass sich die Kontaktmittel in der von außen zugänglichen Kavität befinden, während das restliche Substrat vollständig vom isolierenden Material des Anschlusskörpers 22 umgeben ist. Die elektrischen Kontaktmittel 42 sind mittels eines Fügeverfahrens elektrisch leitend mit der oder den Leiterbahnstrukturen 41 verbunden. Ein solches Fügeverfahren umfasst nicht abschließend eine gelaserte Verbindung, Verschweißung, Verlötung oder Verklebung mit einem elektrischen leitfähigen Klebstoff.

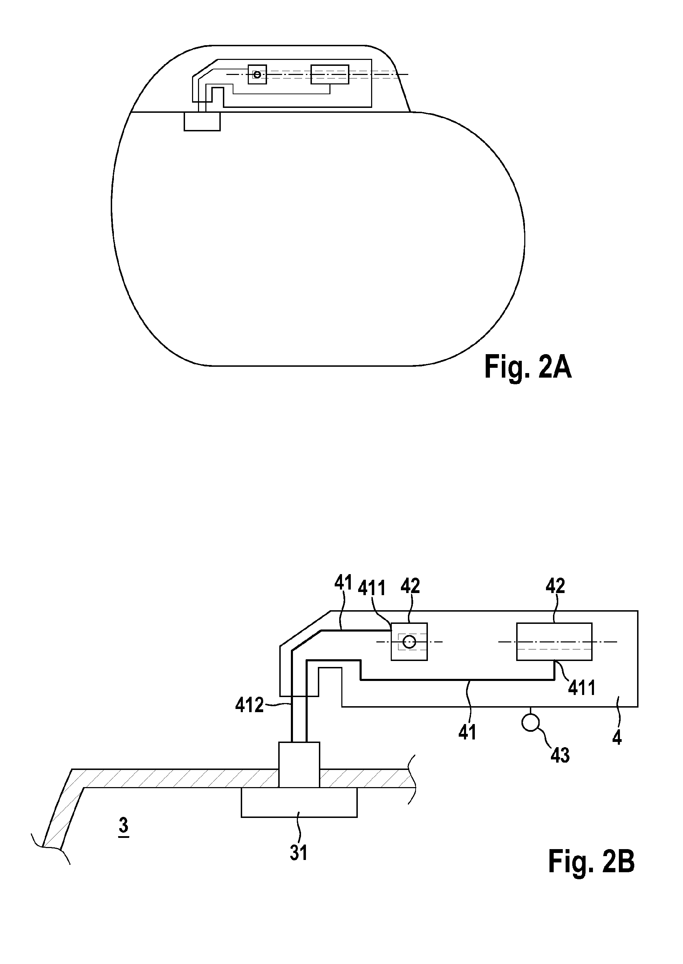

Fig. 1B und Fig. 2B zeigen in Detailansichten eines Substrates 4 gemäß zweier Ausführung, dass eine oder mehrere der Leiterbahnenstrukturen 41 des Anschlussgehäuses 2 ein erstes Ende 411, an welchem die Kontaktmittel 42 elektrisch fest verbunden sind, und ein zweites Ende 412 aufweisen. Jedes der zweiten Enden 412 der Leiterbahnenstrukturen 41 sind mit einem der mindestens einen Durchführungspins elektrisch verbunden. Dabei sind die Durchführungspins der Durchführung 31 und die Leiterbahnstruktur 41 miteinander verlötet, verschweißt, verklebt oder zusammengesteckt und bilden dadurch eine elektrische Verbindung.
Die Kontaktmittel 42 umfassen vorzugsweise aus dem Stand der Technik bekannte Steckeraufnahmen oder Federhülsen Sie können aber auch andere geeignete Formen zur Aufnahme eines Steckers aufweisen, die beispielsweise bei der Einführung neuer Steckerstandards wie IS-4 notwendig werden.
Weiterhin bevorzugt kann das Anschlussgehäuse 2 und/oder das Substrat 4 zusätzliche Verankerungsmöglichkeiten 43 aufweisen, um die Befestigung des Anschlussgehäuses 2 am Hohlgehäuse 3 zu verbessern.

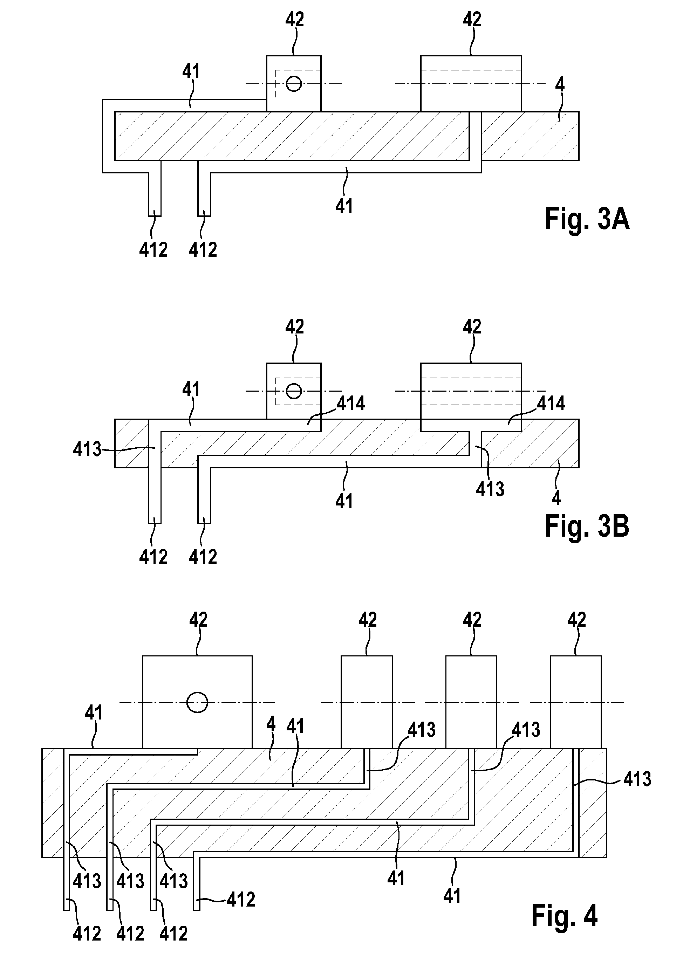

Im Folgenden wird das Substrat 4 näher beschrieben. Fig. 3A zeigt eine Schnittansicht des Substrates 4, das als Leiterplatte (Platine Printed Circuit Board (PCB), gedruckte Schaltung oder Leiterkarte) ausgelegt ist. Die mindestens eine Leiterbahnstruktur 41 des Substratmaterials ist auf dem Substratmaterial 4 angebracht oder vorzugsweise so in das Substratmaterial 4 eingelassen, dass eine vom Substrat 4 weg weisende Leiterbahnfläche nicht vom Substratmaterial bedeckt ist. Im ersteren Fall kann die Leiterbahnstruktur 41 eine so genannte Außenverdrahtung sein, bei der die Leiterbahnstrukturen 41 auf dem dem Substrat aufliegen. Das Substrat dient dabei lediglich zur mechanischen Befestigung der Kontaktmittel 42, beispielsweise mittels Kleben. Die elektrische Verbindung geschieht durch die auf dem Substrat 4 aufliegenden Leiterbahnstrukturen. Wahlweise können die Kontaktmittel 42 auch mittels eines Bonddrahtes als Leiterbahnstruktur mit der Durchführung 31 des Hohlgehäuses 3 elektrisch verbunden sein.
Im zweiten Fall, der in Fig. 3B als Schnittdarstellung eines Substrates 4 dargestellt ist, werden herkömmliche ein- oder zweilagige Leiterplatten verwendet, bei denen Leiterbahnstrukturen 41 durch Ätzverfahren vom isolierenden Substratmaterial befreit sind. Dabei sorgen Durchkontaktierungen (Vias) für die notwendigen elektrischen Verbindungen. Die Leiterbahnstrukturen 41 sind also einseitig unisoliert im Substratmaterial des Substrates 4 eingebettet und mit dem Substratmaterial fest verbunden. Bei dieser Ausführung ist sowohl eine mechanische als auch eine elektrische Verbindung mit dem Substrat 4 vorhanden, da die elektrischen Kontaktmittel 42 auf Lötfahnen oder Pads 414 elektrisch und mechanisch durch die genannten Fügeverfahren verbunden sind. Diese Ausführungsform eignet sich besonders zur automatisierten Vormontage der Substrate mittels SMD-Techniken (Surface Mounted Devices).
Erfindungsgemäß ist das Substratmaterial 4 für das Anschlussgehäuse 2 aus einem starren Material wie faserverstärkten Kunststoffen, Keramik, flammhemmenden Substraten aus Papier wie FR1-/FR2-Substrate (Phenolharz und Papier) oder FR3-Substrate (Epoxidharz und Papier), flammhemmenden Substraten aus Glasfasergewebe wie, FR4-/FR5-Substrate (Epoxidharz und Glasfasergewebe, BT-Substraten, CE- Substraten, Epoxidharz-Laminaten wie CEM1- oder CEM3-Substrate PD-Substraten, PTFE-Substraten (Polytetrafluorethylen), CHn- Substraten, Polyimidfolien (Kaptonfolien), Polyester, oder HDI-Leiterplatten (High-Density-Interconnect-Leiterplatte) hergestellt, um so die notwendige Biegestabilität zum Schutz der Verbindungen zum Hohlgehäuse 3 zu gewährleisten. Zur Gewichtsersparnis und zur Miniaturisierung kann das Substratmaterial 4 aber auch aus starrflexiblen und flexiblen Materialien beispielsweise in Form von Leiterplatten / Platinen oder Folien hergestellt sein. In diesem Fall muss jedoch sichergestellt sein, dass geeignete Versteifungsmittel wie Rippen für die notwendige Stabilität des Anschlussgehäuses 2 sorgen.

Wie in Fig 4 in einer Schnittdarstellung eines mehrlagigen Substrates zu sehen ist, kann alternativ oder zusätzlich zu der soeben genannten Ausführung des Anschlussgehäuses 2 mindestens eine der Leiterbahnstrukturen 41 so in das Substrat 4 eingelassen sein, dass sie vollständig vom Substratmaterial umgeben ist. Im Substratmaterial sind Durchbrüche (Durchkontaktierungen, Vias, Micro-Vias, Buried-Vias) 413 vorgesehen, um die Kontaktmittel 42 mit einer oder mehreren der mindestens einen eingebetteten Leiterbahnstruktur elektrisch fest zu verbinden. Diese Ausführung als mehrlagiges Substrat ist vor allem bei bi- oder mehrpoligen Anschlussgehäusen, die Kavitäten und Kontaktelemente für mehr als eine Elektrodenleitung aufweisen (beispielsweise zur kardialen Resynchronisation aller vier Herzkammern oder zur komplexen Nerven- oder "deep brain stimulation"), oder bei komplexen Steckverbindungen der IS-4-Norm - wie in Fig. 4 dargestellt - vorgesehen.

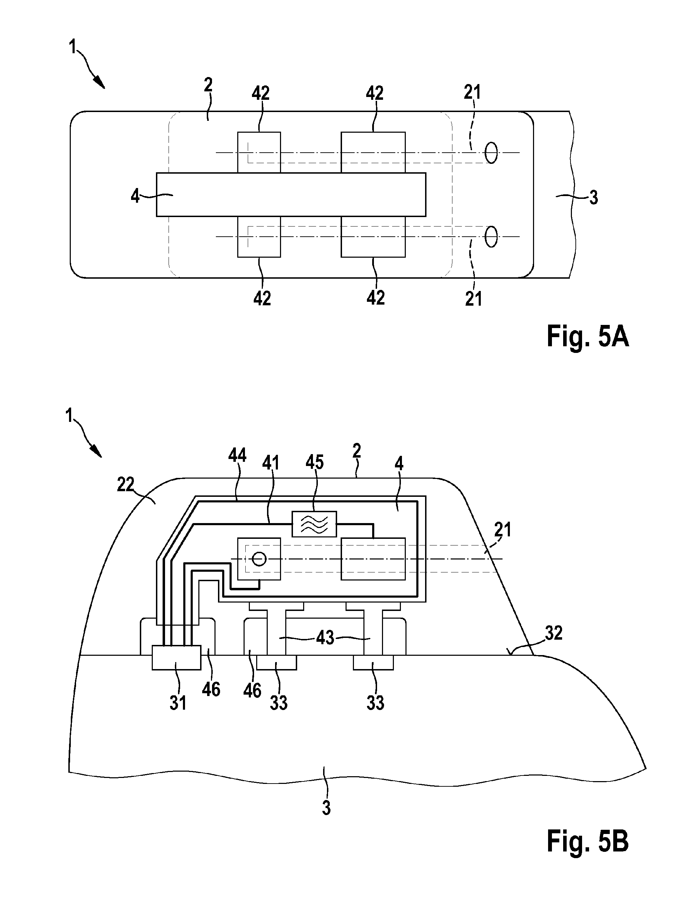

In Fig. 5A und Fig. 5B ist eine Seiten- und eine Draufsicht eines medizinische Implantates 1 gemäß einer weiteren Ausführung dargestellt, bei dem die Leiterbahnstrukturen des Substrates 4 nicht nur zur elektrischen Verbindung der Schaltung im Inneren des Hohlgehäuses 3 dienen können. In Fig. 5A und Fig. 5B ist ein Anschlussgehäuse 2 für ein medizinisches Implantat 1 zu sehen, welches für die bipolare Stimulation vorgesehen ist. Es umfasst in einem Anschlusskörper 22 zwei von außen zugängliche Kavitäten 21, in die jeweils ein Stecker am proximalen Ende einer Elektrodenleitung eingeführt werden kann und dadurch durch die Kontaktmittel 42 in elektrischen Kontakt mit den Leiterbahnstrukturen 41 und damit über den Pin der Durchführung 31 mit der Schaltung im Inneren des Hohlgehäuses 3 gebracht werden. Der Einfachheit wegen sind die beiden Kavitäten 21 nebeneinander angeordnet. Sie können jedoch auch übereinander in Bezug zur Lage des Hohlgehäuses 3 angebracht sein. Die Kavitäten liegen beidseits des Substrats 4. Ebenfalls in den Anschlusskörper 22 eingebettetet ist ein aus isolierendem Material bestehendes Leiterplattensubstrat 4 mit mindestens einer elektrisch leitenden Leiterbahnstruktur 41. Ein oder mehrere Kontaktmittel 42, sind mit einer oder mehreren der mindestens einen in oder auf dem Substratmaterial des Substrats 4 befindlichen Leiterbahnstrukturen 41 fest elektrisch leitend verbunden. Die Kontaktmittel 42 sind dabei fest mit dem Substrat 4 elektrisch und mechanisch verbunden. Das Substrat 4 wiederum ist so in das Anschlussgehäuse eingegossen, dass die Kontaktmittel 42 so in den Kavitäten liegen, dass der Elektrodenstecker sicher mit diesen in Kontakt gebracht werden kann, während das restliche Substrat 4 vollständig vom isolierenden Material des Anschlussgehäuses 22 umgeben ist. Weiterhin umfasst das Substrat 4 mindestens eine Leiterbahnstruktur 44, die als Antennenstruktur ausgeführt ist. Diese Antennenstruktur weist ein erstes und ein zweites Ende auf, welche gelaserte, gelötete, geklebte oder gesteckte Verbindungsstellen zur festen elektrischen und mechanischen Verbindung mit dem Hohlgehäuse 3 umfassen. Die elektrische Verbindung besteht jeweils mit einem Pin der Gehäusedurchführung 31 des Hohlgehäuses. Das Substrat 4 weist zusätzlich zwei Anker 43 auf, welche dazu vorgesehen sind, die Verbindung zwischen Anschlussgehäuse 2 und Hohlgehäuse 3 zu stabilisieren. Die Anker sind entweder durch Substratmaterial geformt und bilden damit ein Gefüge mit dem Stubstrat 4 oder werden geeignet mechanisch (durch Lasern, Schweißen, Löten, Kleben) fest mit dem Substrat verbunden.

In jedem Fall umgibt der Anschlusskörper 22 des Anschlussgehäuses 2 das Substrat 4 und die mindestens eine Leiterbahnstruktur 41 zumindest abschnittsweise so, dass die Verbindungsstellen am zweiten Ende 412 und die dem Hohlgehäuse 3 zugewandten Enden der Anker 43 von außen zugänglich sind, um so das Verbinden mit dem Hohlgehäuse 3 zu ermöglichen. Der Anschlusskörper weist demnach seitlich zugängliche Kavitäten 46 auf, durch die die Verbindung durch die oben genannten Fügeverfahren durchgeführt werden kann. Gemäß einer bevorzugten Ausführung ist das Substrat 4 und die mindestens eine Leiterbahnstruktur 41 jedoch vom Anschlusskörper vollständig umschlossen.

Gemäß einer zusätzlichen Ausführung kann eine weitere vom Anschlusskörpermaterial unterschiedliche Passivierungs- bzw. Isolationsschicht vorgesehen sein, welche unmittelbar auf das Substrat 4 aufgebracht ist und eine zusätzliche (beispielsweise hochspannungsisolierende) Zwischenschicht zwischen Anschlussgehäuse 22 und Substrat 4 bildet. Diese Passivierungs- bzw. Isolationsschicht kann aus organischen bzw. anorganischen Werkstoffen und deren Gemischen (beispielsweise Keramiken oder Polymere) bestehen.

Zur Verankerung des Ankers 43 weist das Hohlgehäuse 3 eine Montagefläche 32 auf, an welcher das Anschlussgehäuse 2 fest verbunden ist. Die Montagefläche 32 umfasst dazu neben der Durchführung 31 Vertiefungen 33, welche geeignet sind, die Verankerungen oder Anker 43, welche das Substrat 4 aufweist, aufzunehmen und dadurch das Anschlussgehäuse 2 in Bezug auf das Hohlgehäuse 3 zu positionieren und zu befestigen.

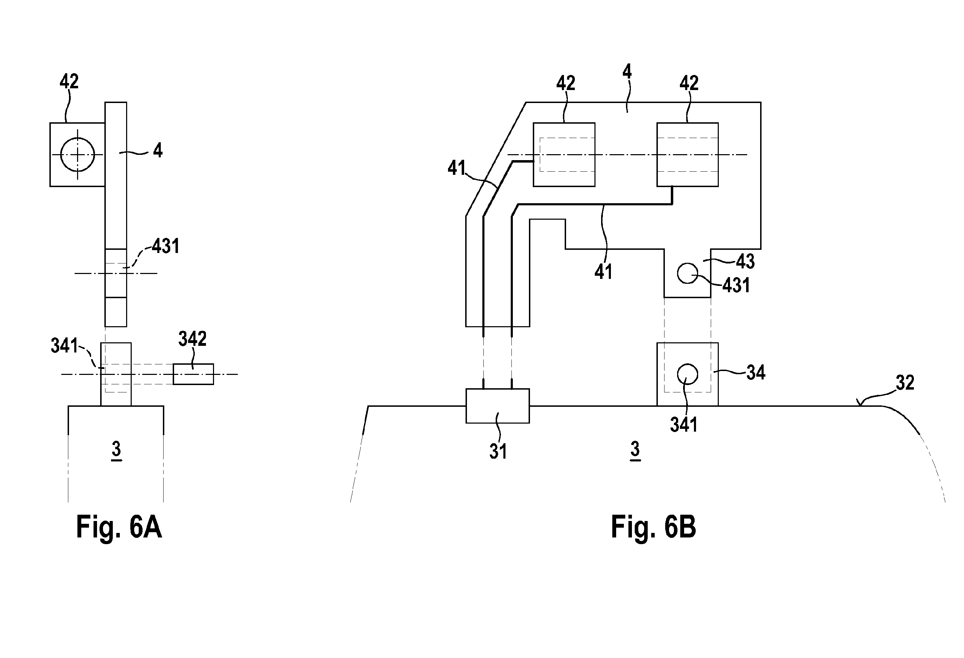

Gemäß einer weiteren, in den Fig. 6A und 6B dargestellten, Ausführung der Verankerung mit dem Hohlgehäuse 3 können sich im Substrat 4 eine oder mehrere Befestigungsmittel 43 wie beispielsweise Ausbuchtungen mit Bohrungen oder Öffnungen befinden, die für eine Befestigung - beispielsweise durch Verstiften - mit am Hohlgehäuse 3 fest verschweißten Verankerungsmittel 34 dienen. Diese Ausbuchtungen des Befestigungsmittels 43 sind aus dem Substratmaterial selbst gebildet und können beispielsweise die Form eines abstehenden Fähnchens aufweisen. Die Verankerungsmittel 34 sind fest auf der Montagefläche 32 befestigte steife Anker mit Aufnahmen in Form einer einseitig von der dem Hohlgehäuse entgegen gesetzten Richtung zugänglichen Aussparung, welche ausgebildet ist, die Befestigungsmittel 43 des Substrats 4 aufzunehmen. Sowohl die Verankerungsmittel 34 als auch die Befestigungsmittel 43 weisen senkrecht relativ zueinander liegende Bohrungen oder Öffnungen 341 und 431 auf, durch welche ein Stift 342 zur Verstiftung geführt werden kann. Bei der Herstellung werden die Befestigungsmittel des Substrats 4 von der dem Hohlgehäuse 3 entgegen gesetzter Richtung in die Ankeraufnahmen gesteckt und mit oder ohne Anschlusskörper 22 mit dem Hohlgehäuse 3 verstiftet. Anschließend kann die Verankerung bei Umspritzen oder Umgießen (Vakkumverguss bzw. Atmosphärenverguß) des Anschlusskörpers zusätzlich mit Kunststoff oder Klebstoff stabilisiert werden.

Wie Fig. 5B weiter zu entnehmen ist, können auf oder im Substrat 4 Filtermittel 45 vorgesehen werden, welche eine Abschirmung der im Hohlgehäuse 3 befindlichen Schaltung gegen elektromagnetische Störgrößen bewirken. Bei dem Filtermittel 45 kann es sich beispielsweise um Hoch-, Tief- oder Bandpassfilter handeln. Es können auch mehrere Filtermittel vorhanden sein. Beispielhaft dargestellt ist ein Filtermittel 45 an einer Leiterbahnstruktur 41 zwischen Kontaktmittel 42 und der elektrischen Verbindung der Leiterbahnstruktur am zweiten Ende 412 mit der Durchführung 31 im Hohlgehäuse 3. Die für das Filtermittel notwendigen passiven Bauteile auf dem Substrat können beispielsweise einfach und automatisiert durch SMD-Technologie bestückt sein. Um Gewicht und Bauteile sowie deren Bestückung zu sparen, können die passiven Bauelemente in das Substrat 4 mehrlagig integriert sein. Induktivitäten, Spulen, kleine Kapazitäten, Kontakte oder Kühlkörper können direkt als Kupferschicht-Struktur ausgebildet werden. Widerstände können mittels spezieller Pasten auf die Oberfläche oder in die verdeckten Layer eines mehrlagigen Substrats 4 eingedruckt werden.

Das Substrat 4 kann an geeigneter Stelle eine oder mehrere Aussparungen für ein oder mehrere röntgendichte Markerelemente aufweisen. Diese Markerelemente sind elektrisch isoliert in der Aussparung befestigt und enthalten Informationen zur Betriebsart und zum Typ des Implantats. Die Informationen werden bei Durchleuchten des Körpers mit Röntgenstrahlung sichtbar. Im Übrigen können auch andere Identifizierungsmittel im oder am Substrat vorhanden sein, beispielsweise ein RFID-Transponder (Radio Frequency Identification).

### Bezugszeichenliste

- 1: medizinisches Implantat
- 2: Anschlussgehäuse
- 3: Hohlgehäuse
- 4: Substrat
- 21: von außen zugängliche Kavität
- 22: Anschlusskörper
- 31: Glas- / Keramikdurchführung
- 32: Montagefläche
- 33: Vertiefungen zur Aufnahme der Verankerungen / Anker am Stubstrat
- 34: Verankerung / Anker am Hohlgehäuse
- 341: Bohrung/Öffnung in der Verankerung am Hohlgehäuse
- 342: Stift
- 41: Leiterbahnstruktur
- 42: Kontaktmittel
- 43: Verankerungen / Anker am Substrat
- 431: Bohrung/Öffnung in der Verankerung am Hohlgehäuse
- 44: Antennenstrutkur
- 45: Filtermittel
- 46: seitlich zugängliche Kavitäten, durch welche die Verbindung zwischen Leiterbahnstruktur und Durchführungspin sowie zwischen Verankerung / Anker und Hohlgehäuse erfolgen kann
- 411: erste Enden der Leiterbahnstruktur, an der die Kontaktmittel elektrisch und mechanisch verbunden sind
- 412: zweite Enden der Leiterbahnstruktur, an welchem eine elektrische und mechanische Vebindung zur Glas- / Keramikdurchführung besteht
- 413: Durchbruch / Via im Substrat
- 414: Lötfahnen oder Pads auf dem Substrat

## Patentansprüche

1. Anschlussgehäuse (2) für ein medizinisches Implantat (1) umfassend einen Anschlusskörper (22) aus elektrisch isolierendem Material, der wenigstens eine von außen zugängliche Kavität (21) zum Anschließen einer implantierbaren Elektrodenleitung besitzt,
Kontaktmittel (42), die sich in der von außen zugänglichen Kavität befinden; und
ein aus isolierendem Material bestehendes plattenförmiges Substrat (4) mit mindestens einer elektrisch leitenden Leiterbahnstruktur (41, 44), wobei die Kontaktmittel (42) mit einer oder mehreren der mindestens einen in oder auf dem Substratmaterial des Substrats befindlichen Leiterbahnstruktur (41) fest elektrisch leitend verbunden sind,
**dadurch gekennzeichnet, dass**
die Kontaktmittel (42) auf dem Substrat angeordnet und mit dem Substrat mechanisch fest verbunden sind; und
das Substrat so in den Anschlusskörper eingebettet ist, dass sich die Kontaktmittel in der von außen zugänglichen Kavität befinden.

2. Anschlussgehäuse nach Anspruch 1, **dadurch gekennzeichnet, dass** eine oder mehrere der mindestens einen Leiterbahnstruktur (41) ein erstes Ende (411), an welchem je eines der Kontaktmittel (42) elektrisch fest verbunden ist, und ein zweites Ende (412) aufweist, welches Verbindungsstellen zur festen elektrischen Verbindung mit einem Hohlgehäuse (3) umfasst, wobei jedes der Kontaktmittel (42) vorzugsweise Steckeraufnahmen oder Federhülsen umfasst.

3. Anschlussgehäuse nach Anspruch 1 oder 2, **dadurch gekennzeichnet, dass** die mindestens eine Leiterbahnstruktur (41) auf dem Substrat (4) angebracht oder vorzugsweise so in das Substratmaterial eingelassen ist, dass eine vom Substrat weg weisende Leiterbahnfläche nicht vom Substratmaterial bedeckt ist.

4. Anschlussgehäuse nach Anspruch 1 oder 2, **dadurch gekennzeichnet, dass** die mindestens eine Leiterbahnstruktur (41) so in das Substrat (4) eingelassen ist, dass sie vollständig vom Substratmaterial umgeben ist, und dass im Substratmaterial Durchbrüche (413) vorgesehen sind, um Kontaktmittel (42) mit einer oder mehreren der mindestens einen Leiterbahnstruktur elektrisch fest zu verbinden.

5. Anschlussgehäuse nach einem der Ansprüche 1 bis 4, **dadurch gekennzeichnet, dass** die Kontaktmittel (42) mit der mindestens einen Leiterbahnstruktur (41) durch Schweißen, Löten oder Kleben elektrisch fest verbunden sind.

6. Anschlussgehäuse nach Anspruch 1, **dadurch gekennzeichnet, dass** eine der mindestens einen Leiterbahnstruktur als Antennenstruktur (44) ausgeführt ist und wobei die Antennenstruktur ein erstes und ein zweites Ende aufweist, welche Verbindungsstellen zur festen elektrischen Verbindung mit dem Hohlgehäuse (3) umfassen.

7. Anschlussgehäuse nach einem der Ansprüche 1 bis 6, **dadurch gekennzeichnet, dass** das Substrat Verankerungen (43) aufweist, welche zur Befestigung des Anschlussgehäuses an das Implantat dienen.

8. Anschlussgehäuse nach Anspruch 6 und 7, **dadurch gekennzeichnet, dass** der Anschlusskörper (22) das Substrat (4) und die mindestens eine Leiterbahnstruktur (41) zumindest abschnittsweise so umgibt, dass die Verbindungsstellen und die Verankerung (43) von außen zugänglich sind, vorzugsweise sind das Substrat und die mindestens eine Leiterbahnstruktur jedoch vom Anschlusskörper vollständig umschlossen.

9. Anschlussgehäuse nach einem der Ansprüche 1 bis 8, **dadurch gekennzeichnet, dass** das Substrat (4)als (Schaltkreis-)Platine ausgelegt ist, wobei das Substratmaterial bevorzugt faserverstärkten Kunststoffen, Keramik, Epoxyd-Glasgewebe (FR-4 Substrat), BT-Substrat, CE- Substrat, CEM1-Substrat, CEM3-Substrat, FR-2 Substrat, FR-3 Substrat, FR-5 Substrat, PD-Substrat, PTFE-Substrat, CHn- Substrat, Polyimidfolien (Kapton) oder Polyester ist, und wobei die Leiterbahnstruktur (41) draht- oder bandförmig ist und besonders bevorzugt aus Kupfer, Aluminium, Gold, Silber, Edelstahl, Titan, Niob, Tantal, leitfähigen Klebstoff bzw. Kunststoff geätzt oder auf das Substrat werden ausgestanzte, geätzte Leiterbahnstrukturen auf das Substrat geklebt. besteht.

10. Anschlussgehäuse nach einem der Ansprüche 1 bis 9, **dadurch gekennzeichnet, dass** das Substrat (4) eine oder mehrere Aussparungen für ein oder mehrere Markerelemente aufweist, wobei jedes Markerelement elektrisch isoliert in der Aussparung befestigt ist.

11. Verfahren zur Herstellung eines Anschlussgehäuses (2) für ein medizinisches Implantat nach einem der Ansprüche 1 bis 10, umfassend einen Anschlusskörper (22) aus elektrisch isolierendem Material, der wenigstens eine von außen zugängliche Kavität (21) zum Anschließen einer implantierbaren Elektrodenleitung besitzt, mit den folgenden Schritten:
- Fixieren eines elektrischen Substrats (4) mit darauf elektrisch fest verbundenen Kontaktmitteln (42) in einer Spritzgussform,
- Spritzvorgang/Umspritzvorgang des Anschlusskörpers so, dass das Substrat so in den Anschlusskörper durch Umspritzen oder Umgießen so eingebettet ist, dass sich die Kontaktmittel in der von außen zugänglichen Kavität befinden.

12. Medizinisches Implantat, umfassend:
- ein Hohlgehäuse (3), welches hermetisch abgedichtet ist und mindestens eine hermetische Durchführung (31) aufweist, welche geeignet ist, elektrische Signale durch das Hohlgehäuse hindurch zu leiten, ohne dessen Hermetizität zu beeinträchtigen, wobei die hermetische Durchführung mindestens einen Durchführungspin aufweist,
- eine im Hohlgehäuse befindliche Schaltung, wobei die Schaltung geeignet ist, Signale des menschlichen Körpers zu messen und Signale an den menschlichen Körper abzugeben und die geeignet ist, Signale an einen außerhalb des Körpers liegenden Empfänger zu senden, und
- ein Anschlussgehäuse (2) nach einem der Ansprüche 1 bis 10, welches fest mit dem Hohlgehäuse verbunden ist,
**dadurch gekennzeichnet, dass**
jeder der mindestens einen Durchführungspins mit einer der mindestens einen elektrisch leitenden Leiterbahnstrukturen (42) elektrisch verbunden ist.

13. Medizinisches Implantat nach Anspruch 12, **dadurch gekennzeichnet, dass** das Hohlgehäuse (3) eine Montagefläche (32) aufweist, an welcher das Anschlussgehäuses (2) fest verbunden ist und welche Vertiefungen (33) umfasst, welche geeignet sind, die Verankerungen (43), welche das Substrat (4) aufweist, aufzunehmen und dadurch das Anschlussgehäuse in Bezug auf das Hohlgehäuse zu positionieren und zu befestigen.

14. Medizinisches Implantat nach Anspruch 12, **dadurch gekennzeichnet, dass** jeder der mindestens eine Durchführungspin mit einer der mindestens einen elektrisch leitenden Leiterbahnstruktur (41) gelötet, geschweißt, geklebt oder gesteckt ist.

## Claims

1. A connection housing (2) for a medical implant (1), comprising a connection body (22), which is made of electrically insulating material and which has at least one externally accessible cavity (21) for connecting an implantable electrode line, contact means (42), which are located in the externally accessible cavity; and
a plate-shaped substrate (4) consisting of insulating material and having at least one electrically conductive strip conductor structure (41, 44), wherein the contact means (42) are securely electrically conductively connected to one or more of the at least one strip conductor structures (41) present in or on the substrate material of the substrate,
**characterised in that**
the contact means (42) are arranged on the substrate and are securely mechanically connected to the substrate; and
the substrate is embedded in the connection body such that the contact means are present in the externally accessible cavity.

2. The connection housing according to Claim 1, **characterised in that** one or more of the at least one strip conductor structures (41) has a first end (411) to which one of the contact means (42) is securely electrically connected in each case, and a second end (412) which comprises connecting sites for the secure electrical connection to a hollow housing (3), wherein each of the contact means (42) preferably comprises plug receptacles or spring sleeves.

3. The connection housing according to Claim 1 or 2, **characterised in that** the at least one strip conductor structure (41) is mounted on the substrate (4) or is preferably embedded in the substrate material such that a strip conductor surface facing away from the substrate is not covered by the substrate material.

4. The connection housing according to Claim 1 or 2, **characterised in that** the at least one strip conductor structure (41) is embedded in the substrate (4) such that said strip conductor structure is surrounded fully by the substrate material, and **in that** passages (413) are provided in the substrate material to securely electrically connect contact means (42) to one or more of the at least one strip conductor structures.

5. The connection housing according to one of Claims 1 to 4, **characterised in that** the contact means (42) are securely electrically connected to the at least one strip conductor structure (41) by welding, soldering, or gluing.

6. The connection housing according to Claim 1, **characterised in that** one of the at least one strip conductor structures is designed as an antenna structure (44), and wherein the antenna structure has a first and a second end which comprise connecting sites for the secure electrical connection to the hollow housing (3).

7. The connection housing according to one of Claims 1 to 6, **characterised in that** the substrate has fasteners (43), which are used for fastening the connection housing to the implant.

8. The connection housing according to Claim 6 and 7, **characterised in that** the connection body (22) surrounds the substrate (4) and the at least one strip conductor structure (41) at least in portions, such that the connecting sites and the fasteners (43) are externally accessible, preferably the substrate and the at least one strip conductor structure are surrounded fully by the connection body.

9. The connection housing according to one of Claims 1 to 8, **characterised in that** the substrate (4) is designed as a (circuit) board, wherein the substrate material is preferably fibre-reinforced plastics, ceramic, epoxy glass fabric (FR-4 substrate), BT substrate, CE substrate, CEM1 substrate, CEM3 substrate, FR-2 substrate, FR-3 substrate, FR-5 substrate, PD substrate, PTFE substrate, CHn substrate, polyimide films (Kapton), or polyester, and wherein the strip conductor structure (41) is wire-shaped or ribbon-shaped and particularly preferably consists of copper, aluminium, gold, silver, stainless steel, titanium, niobium, tantalum, conductive adhesive, or plastic etched onto the substrate, or punched-out, etched strip conductor structures are glued onto the substrate.

10. The connection housing according to one of Claims 1 to 9, **characterised in that** the substrate (4) has one or more recesses for one or more marker elements, wherein each marker element is fastened in the recess in an electrically insulated manner.

11. A method for producing a connection housing (2) for a medical implant according to one of Claims 1 to 10, comprising a connection body (22), which is made of electrically insulating material and which has at least one externally accessible cavity (21) for connecting an implantable electrode line, said method having the following steps:
- fixing an electrical substrate (4) with contact means (42) securely electrically connected thereto in an injection mould,
- injection moulding/overmoulding the connection body such that the substrate is embedded in the connection body by overmoulding or encapsulation, such that the contact means are located in the externally accessible cavity.

12. A medical implant, comprising:
- a hermetically sealed hollow housing (3) and at least one hermetic feedthrough (31) suitable for guiding electrical signals through the hollow housing without impairing the hermeticity thereof, wherein the hermetic feedthrough has at least one feedthrough pin,
- a circuit located in the hollow housing, wherein the circuit is suitable for measuring signals of the human body and delivering signals to the human body and is suitable for sending signals to a receiver located outside the body, and
- a connection housing (2) according to one of Claims 1 to 10, which is securely connected to the hollow housing,
**characterised in that**
each of the at least one feedthrough pins is electrically connected to one of the at least one electrically conductive strip conductor structures (42).

13. The medical implant according to Claim 12, **characterised in that** the hollow housing (3) has a mounting surface (32), to which the connection housing (2) is securely connected and which comprises depressions (33) that are suitable for accommodating the fasteners (43) comprised by the substrate (4) and thus for positioning and fastening the connection housing with respect to the hollow housing.

14. The medical implant according to Claim 12, **characterised in that** each of the at least one feedthrough pins is soldered, welded, glued, or mounted to one of the at least one electrically conductive strip conductor structures (41).

## Revendications

1. Boîtier de raccordement (2) pour un implant médical (1) comprenant un corps de raccordement (22) constitué d'un matériau isolant de l'électricité, qui possède au moins une cavité (21) accessible par l'extérieur pour le raccordement d'une ligne d'électrode implantable,
des moyens de contact (42) qui se situent dans la cavité accessible par l'extérieur ; et un substrat (4) en forme de plaque constituée d'un matériau isolant avec au moins une structure de pistes conductrices (41, 44) conduisant l'électricité, dans lequel les moyens de contact (42) sont reliés solidement en conduisant l'électricité avec un ou plusieurs des au moins une structure de pistes conductrices (41) se trouvant dans, ou sur le matériau de substrat du substrat,
**caractérisé en ce que**
les moyens de contact (42) sont disposés sur le substrat et sont solidement reliés mécaniquement avec le substrat ; et
le substrat est incorporé dans le corps de raccordement de telle manière que les moyens de contact se situent dans la cavité accessible par l'extérieur.

2. Boîtier de raccordement selon la revendication 1, **caractérisé en ce qu'**une ou plusieurs parmi les au moins une structure de piste conductrice (41) présente une première extrémité (411) à laquelle au moins un des moyens de contact (42) est relié électriquement de manière solide, et une deuxième extrémité (412) laquelle comprend des points de liaison pour la liaison électrique solide avec un boîtier creux (3), dans lequel chacun parmi les moyens de contact (42) comprend de préférence des fiches de raccordement ou des douilles à ressort.

3. Boîtier de raccordement selon les revendications 1 ou 2, **caractérisé en ce que** l'au moins une structure de piste conductrice (41) est rapportée sur le substrat (4) ou de préférence est ainsi immergée dans le matériau du substrat qu'une surface de piste conductrice s'éloignant du substrat n'est pas recouverte par le matériau du substrat.

4. Boîtier de raccordement selon les revendications 1 ou 2, **caractérisé en ce que** l'au moins une structure de piste conductrice (41) est ainsi immergée dans le substrat (4) qu'elle est totalement entourée par le matériau du substrat et que des passages (413) sont prévus dans le matériau du substrat afin de relier électriquement de manière solide des moyens de contact (42) avec une ou plusieurs parmi les au moins une structure de piste conductrice.

5. Boîtier de raccordement selon l'une des revendications 1 à 4, **caractérisé en ce que** les moyens de contact (42) sont reliés électriquement de manière solide avec au moins une structure de piste conductrice (41) par soudure, brasage ou collage.

6. Boîtier de raccordement selon la revendication 1, **caractérisé en ce qu'**une des au moins une structure de piste conductrice est conçue sous la forme d'une structure d'antenne (44) et dans lequel la structure d'antenne présente une première et une deuxième extrémités, lesquelles comprennent des points de liaison pour une liaison électrique solide avec le boîtier creux (3).

7. Boitier de raccordement selon l'une des revendications 1 à 6, **caractérisé en ce que** le substrat présente des ancrages (43), lesquels servent à la fixation du boîtier de raccordement à l'implant.

8. Boîtier de raccordement selon les revendications 6 ou 7, **caractérisé en ce que** le corps de raccordement (22) entoure le substrat (4) et l'au moins une structure de piste conductrice (41) au moins de manière partielle de sorte que les points de liaison et les ancrages (43) soient accessibles par l'extérieur, de préférence, le substrat et l'au moins une structure de piste conductrice sont pourtant totalement enfermés dans le corps de raccordement.

9. Boîtier de raccordement selon l'une des revendications 1 à 8, **caractérisé en ce que** le substrat (4) est élaboré en tant que platine de circuit intégré, dans lequel le matériau du substrat est de préférence constitué de matières synthétiques renforcées de fibres, de céramique, d'époxy renforcé de fibres de verre (substrat FR-4), de substrat BT, de substrat CE, de substrat CEM1, de substrat CEM3, de substrat FR-2, de substrat FR-3, de substrat FR-5, de substrat PD, de substrat PTFE, de substrat CHn, de feuilles de polyimide (Kapton) ou de polyester, et dans lequel la structure de piste conductrice (41) est en forme de fil ou de bande et de manière préférée, constituée de cuivre, d'aluminium, d'or, d'argent, d'acier inoxydable, de titane, de niobium, de tantale, d'un adhésif conducteur, respectivement, d'une matière plastique gravée, ou des structures de pistes conductrices gravées, estampées sont collées sur le substrat.

10. Boîtier de raccordement selon l'une des revendications 1 à 9, **caractérisé en ce que** le substrat (4) présente un ou plusieurs évidements pour un ou plusieurs éléments marqueurs, dans lequel chaque élément marqueur est fixé dans l'évidement isolé électriquement.

11. Procédé de fabrication d'un boîtier de raccordement (2) pour un implant médical selon l'une des revendications 1 à 10, comprenant un corps de raccordement (22) constitué d'un matériau isolant de l'électricité, qui possède au moins une cavité (21) accessible par l'extérieur pour le branchement d'une ligne d'électrode implantable, avec les étapes suivantes :
- fixation d'un substrat (4) électrique avec des moyens de contact (42) y étant reliés électriquement de manière solide dans un moule d'injection,
- processus d'injection/ processus de surmoulage du corps de raccordement de telle sorte que le substrat est ainsi immergé par un surmoulage ou un entourage que les moyens de contact se trouvent dans la cavité accessible par l'extérieur.

12. Implant médical, comprenant :
- un boîtier creux (3), lequel est étanche hermétiquement et présente au moins un passage (31) hermétique, lequel est approprié pour conduire des signaux électriques à travers le boîtier creux sans détériorer son caractère hermétique, dans lequel le passage hermétique présente au moins une broche de passage,
- un circuit se trouvant dans le boîtier creux, dans lequel le circuit est approprié pour mesurer des signaux du corps humain et pour délivrer des signaux au corps humain et qui est approprié pour émettre des signaux vers un récepteur se situant en dehors du corps, et
- un boîtier de raccordement (2) selon l'une des revendications 1 à 10, lequel est solidement relié avec le boîtier creux,
**caractérisé en ce que**
chacune parmi les au moins une broche de passage est électriquement reliée avec au moins l'une des structures de piste conductrice (42) électriquement conductrice.

13. Implant médical selon la revendication 12, **caractérisé en ce que** le boîtier creux (3) présente une surface de montage (32), à laquelle le boîtier de raccordement (2) est solidement relié et laquelle comprend des cavités (33), lesquelles sont appropriées pour recueillir les ancrages (43) que présente le substrat (4) et, par ce moyen, positionner le boîtier de raccordement par rapport au boitier creux et le fixer.

14. Implant médical selon la revendication 12, **caractérisé en ce que** chacune des au moins une broche de passage est soudée, brasée, collée ou enfichée avec au moins une structure de piste conductrice (41) électriquement conductrice.
